# EUROPÄISCHE PATENTSCHRIFT

(11) **EP 1 499 006 B1**
(45) Veröffentlichungstag und Bekanntmachung des Hinweises auf die Patenterteilung: **02.01.2008**
(21) Anmeldenummer: 04016860.1
(22) Anmeldetag: 16.07.2004
(51) Int. Cl.: H02M 5/32, A61B 5/053

(54) **Vorrichtung zum potentialgetrennten Umwandeln einer ersten Spannung in eine zweite Spannung zum Messen von Impedanzen und Admittanzen an biologischen Geweben**
Device for potentially separated conversion of a first tension into a second tension to measure impedance and admittance of biological tissues
Dispositif pour la conversion potentiellement separée d'une première tension dans une deuxième tension pour mèsurer l'impedance et admittance de tissus biologique

(30) Priorität: 18.07.2003 DE 10332820
(43) Veröffentlichungstag der Anmeldung: 19.01.2005
(73) Patentinhaber: Osypka Medical GmbH, 12277 Berlin (DE)
(72) Erfinder: Gersing, Eberhard, Dr., 37075 Göttingen (DE)
(74) Vertreter: Behrens, Dieter

(56) Entgegenhaltungen:
- WO-A-02/094090
- GB-A- 618 405
- GB-A- 1 172 774
- US-A- 3 402 572

## Beschreibung

Die Erfindung betrifft eine Vorrichtung zum potentialgetrennten Umwandeln einer ersten Spannung in eine zweite Spannung, wie sie, allgemein, bei wirklich floatenden, d.h. nicht auf ein Potential bezogenen Messgeräten, z.B. in der Medizintechnik, und insbesondere im Zusammenhang mit der Messung von Impedanzen und Admittanzen an biologischen Geweben, insbesondere am menschlichen Körper, für die Stromversorgung des oder der elektronischen Messgeräte einsetzbar ist.

Die Impedanz- oder Admittanzmessung an biologischen Geweben, z. B. am menschlichen Körper, ermöglicht Aussagen über deren Zustand, wenn geeignete Frequenzen verwendet werden. Beispielsweise können bei Herzpatienten im Verlauf von und nach Herzoperationen aufgrund der Impedanzmessung für die Diagnose wertvolle Aussagen gewonnen werden. Dasselbe gilt auch bei Organtransplantationen zur Bestimmung der Ischämie-bedingten Schädigung und/oder Wiedererholung des transplantierten Organs.

Für die Messgeräte für elektrische Messungen an inneren Organen des Menschen gelten strenge gesetzliche Sicherheitsvorschriften. Insbesondere muss gewährleistet sein, dass kein Fehlerstrom, der 10 µA überschreitet, über den Menschen abfließt, und dass zwischen dem Netz, aus dem die verwendeten Messgeräte versorgt werden, und dem menschlichen Messobjekt eine Isolationsbarriere besteht, die einem Potentialunterschied von 4 kV_{eff} standhält.

Da das Netz, dem die für die Messgeräte notwendige Energie fast ausschließlich entnommen wird, eine Spannung aufweist, die ohnehin in eine oder mehrere für die verwendeten Messgeräte geeignete(n) Spannung(en) umgewandelt werden muss, bietet es sich an, in einem Schritt bzw. einer einzigen Baugruppe die Umwandlung der einen Spannung in die andere(n) Spannung(en) mit der Potentialtrennung zu kombinieren.

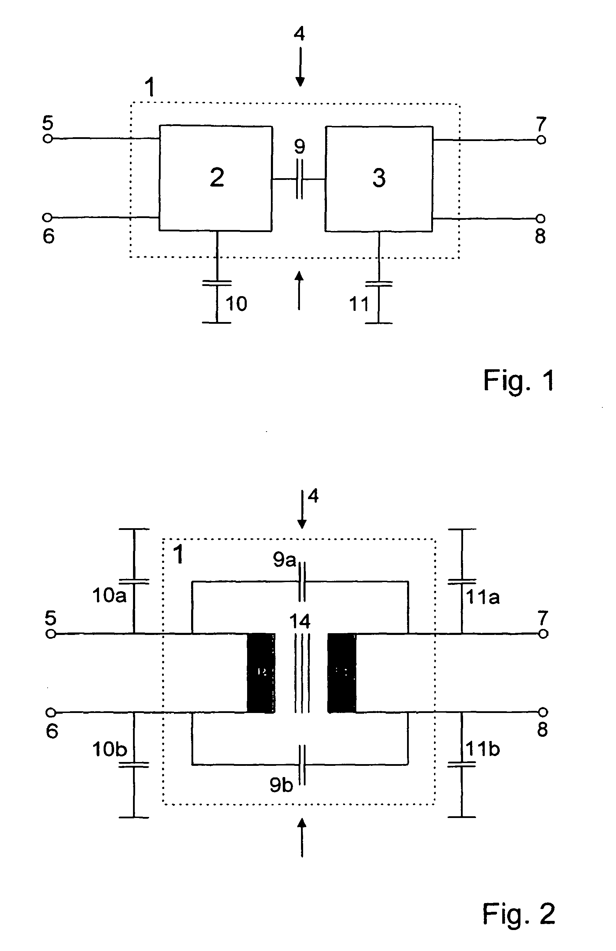

Außer der Potentialtrennung gibt es weitere Anforderungen an eine Vorrichtung zur Stromversorgung eines Impedanzmessgerätes, wie sie in Fig. 1 dargestellt ist. Ein Ausgangskreis 3 einer solchen Vorrichtung 1 mit den Anschlüssen 7 und 8 soll eine möglichst kleine Kapazität 9 gegenüber dem potentialgetrennten Eingangskreis 2, der über die Anschlüsse 5 und 6 mit dem Netz verbunden ist, und eine ebenfalls möglichst kleine Kapazität 11 gegen Erde (Masse) aufweisen. Damit wird gewährleistet, dass das Messgerät auch bei höheren Frequenzen nicht auf Erde bzw. Masse bezogen ist, und folglich die (insbesondere hochfrequenten) Messhilfsströme, die vom Messgerät in den menschlichen Körper eingeleitet werden, nicht über diesen zur Erde abfließen, sondern mit dem Messgerät vollständig erfasst werden. Des weiteren sollen über eine Potentialbarriere 4 keine Störungen (insbesondere HF-Störungen) aus dem Netz auf den Ausgangskreis 3 übertragen werden.

Herkömmlicherweise werden hierfür in solchen Stromversorgungsvorrichtungen Transformatoren eingesetzt, wie dies in Fig. 2 dargestellt ist. Die eingangs genannten Sicherheitsvorschriften sind jedoch sehr streng, und es ist schwierig, die Kriechströme und insbesondere die durch die Kapazität 9a und 9b der Wicklungen 12 und 13 bedingten dielektrischen Verschiebungsströme unter 10 µA zu halten. Durch Erdung des Transformatorkerns 14 und besondere Anordnung der Wicklungen erreicht man zwar eine relativ geringe Kapazität zwischen Primär- und Sekundärwicklung, erhöht jedoch die Kapazitäten 11a und 11b zwischen der Sekundärwicklung 13 und Erde. Ein geschlossener Stromkreis ergibt sich beispielsweise, wenn der Patient, an dem eine Impedanz gemessen werden soll, (wenn auch nur kapazitiv) mit der Erde (Masse) verbunden ist, und die Messgeräte über das Netz, das ebenfalls einen Masseanschluss hat, betrieben werden. Bei diesen kleinen Größenordnungen der Messströme üben die Kapazitäten zwischen den Wicklungen der Primär- und der Sekundärspule der Transformatoren sowie zwischen der Sekundärwicklung und Masse einen erheblichen störenden Einfluss aus. Bisher waren Verbesserungen darauf gerichtet, bei den Transformatoren die Kriechströme zu unterdrücken und die kapazitive Kopplung zu verringern. Nur mit viel Aufwand können die oben genannten Grenzwerte unterschritten werden. Dabei bleibt die Forderung nach geringer Kapazität des Sekundärkreises gegenüber Erde unerfüllt.

Es ist zwar schon eine Vorrichtung zur Messung sowohl der Impedanz als auch des Gleichstrom-Widerstandes der Haut bekannt (WO 00/01301 A1), dabei erfolgt die Stromversorgung des Gerätes aus dem Netz über einen Transformator und die Versorgung der elektronischen Baugruppen für die Impedanzmessung über einen zusätzlichen DC/DC-Wandler. Die elektronisch erzeugte Messwechselspannung wird über einen Transformator (entweder direkt oder nach Gleichrichtung) an die Elektroden angelegt, die mit der Haut in Kontakt sind. Der Strom durch den Messkreis bewirkt einen Spannungsabfall an einem Widerstand, dieser wird verstärkt, gleichgerichtet, und kann über einen ADC gemessen werden, dabei aber nicht mittels eines Stroms - wie bei der Impedanzmessung - sondern mittels einer Wechselspannung aus einer Quelle kleinen Innenwiderstandes über einen Transformator.

Die Erzeugung von Gleich- oder Wechselspannungen unterschiedlicher Größe, Kurvenform, Frequenz und/oder Modulation mittels eines Motor-Drehtransformator-Systems und zwar für therapeutische Zwecke ist bekannt (US Patentschrift 1,908,688). Die erzeugte Spannung wird *direkt* über Elektroden an den menschlichen Körper angelegt. Trotz galvanischer Trennung der Ausgangsseite der Vorrichtung von der Eingangsseite (Netz) über induktive Kopplungen lässt sich die heute geforderte Spannungsfestigkeit von 4 kV_{eff} und ein maximaler Fehlerstrom (Leckstrom) < 10 µA praktisch nicht und eine geringe elektrische Kapazität zwischen Eingangs- und Ausgangsseite schon gar nicht erreichen. Diese Vorrichtung dient ebenso wie die zuvor genannte zur Erzeugung von isolierten Messsignalen zur Anwendung am menschlichen Körper eignet sich aber nicht zur Stromversorgung von Messgeräten.

Die Verwendung einer Motor-Generator-Stromversorgung als "motor generator set" ist in der US Patentschrift 2,327,874 als Möglichkeit zur Versorgung eines portablen elektromedizinischen Gerätes angegeben, jedoch ohne Hinweis auf die speziellen Erfordernisse der elektrische Isolation zwischen Motor und Generator und das Erfordernis einer besonders geringen Kapazität der Generatorseite gegenüber Masse und der Antriebsseite, sowie der Isolation gegenüber elektrischen netzseitigen Störungen und der Überbrückung kurzzeitiger Netzausfälle bei Netzbetrieb.

In EP 0 480 078 A1, die eine Messvorrichtung mit nichtelektrischer Signal- und Energieübertragung an einen entfernten Ort offenbart, ist ein Motor-Generator-System mit isolierter Welle im Bereich der Starkstromtechnik erwähnt, an dem jedoch die "große mechanische Störanfälligkeit" und die Verschleißerscheinungen bei bewegten Teilen bemängelt werden. Über bei der Messtechnik im Bereich der Medizin einzuhaltende kritische Parameter sind naturgemäß keine Angaben gemacht.

Es ist Aufgabe der Erfindung, eine Vorrichtung zum potentialgetrennten Umwandeln einer ersten Spannung, die auf Erde bezogen ist, in eine zweite bereitzustellen, die eine möglichst kleine Kapazität der Quelle der zweiten Spannung gegenüber Erde hat, die Störsignale auf der Eingangsseite nicht auf die Ausgangsseite überträgt und selbst keine zusätzlichen Störungen erzeugt. Diese letztere Bedingung ist bei kommerziell erhältlichen DC/DC- oder AC/DC-Wandlern nicht erfüllt. Sie weisen vielmehr Gleichtakt-Störspannungen auf der Ausgangsseite und beachtliche hochfrequente magnetische Streufelder auf, die auf empfindliche Schaltungsteile eines Messgerätes störend einwirken können. Gleichtaktstörungen können im Prinzip mittels eines Kondensators von entsprechender Kapazität (und Spannungsfestigkeit), der die Massen der Eingangs- und der Ausgangsseite miteinander verbindet, vermindert werden. Diese Maßnahme würde allerdings der für eine einwandfreie Impedanzmessung am Menschen notwendigen Forderung nach möglichst geringer Kapazität (9 in Fig. 1) zwischen Eingangs- und Ausgangsseite des Netzteils direkt zuwiderlaufen.

Die Vorrichtung soll einen einfachen Aufbau haben und somit relativ preisgünstig sein und dennoch zuverlässig die sehr strengen gesetzlichen Sicherheitsvorschriften einhalten, z. B. dass der ausgangsseitige Leckstrom nicht größer als 10 µA wird und dass eine Sicherheitsbarriere von 4 kV_{eff} gewährleistet ist.

Diese Aufgabe ist erfindungsgemäß mit den Vorrichtungen nach Anspruch 1 gelöst. Vorteilhafte Ausgestaltungen ergeben sich aus Unteransprüchen.

Die Erfindung sieht vor, dass zumindest ein Abschnitt der Kopplungsvorrichtung zwischen Motor und Generator aus elektrisch einem isolierendem Kunsthoffmaterial wie Nylon, Trovidur® (Markenname) oder Polystyrol oder einem Keramikmaterial wie Degussit® (Markenname) oder Zirkonoxid besteht. Dabei handelt es sich allgemeinen um eine Welle oder einen Wellenabschnitt, aber auch die Motor- oder die Generatorwelle, oder um z.B. den Riemen eines Riementriebs oder um eine Wellenkupplung, z.B. eine elastische Kupplung, wenn nicht eine durchgehende hochfeste keramische Welle aus insbesondere Zirkonoxid, eingesetzt werden kann. Die Kapazität des Generators gegenüber Masse soll weniger als etwa 10 pF, vorzugsweise 8 pF, insbesondere 5 pF, betragen.

Die zweite Spannung ist im Regelfall eine Niederspannung zum Betrieb elektronischer Schaltungen eines Messgeräts.

In den Ansprüchen wird mit Abschnitt der Welle jede Teileinheit einer Welle in ihrem Längsverlauf gesehen. Ist die Welle beispielsweise eine zylindrische Stange mit konstantem Durchmesser, so ist ein Abschnitt der Welle ein Zylinder, der denselben Querschnitt aufweisen kann, aber nicht ganz so lang wie die Stange ist. Unter einem Abschnitt der Welle ist nicht ein Teilzylinder zu verstehen, der nur einen geringeren Durchmesser als die Welle hat aber nicht aus isolierendem Material besteht.

Durch den elektrisch isolierenden Abschnitt der Welle oder die Ausbildung einer gesonderten Verbindungswelle aus elektrisch isolierendem Material mit einer niedrigen Dielektrizitätskonstante wird eine hohe Potentialbarriere zwischen Motor und Generator ermöglicht, die verhindert, dass Kriechströme vom Motor zum Generator fließen können bzw. umgekehrt. Außerdem wird die kapazitive Kopplung zwischen Motor und Generator durch angemessenen Abstand voneinander ausreichend klein gehalten.

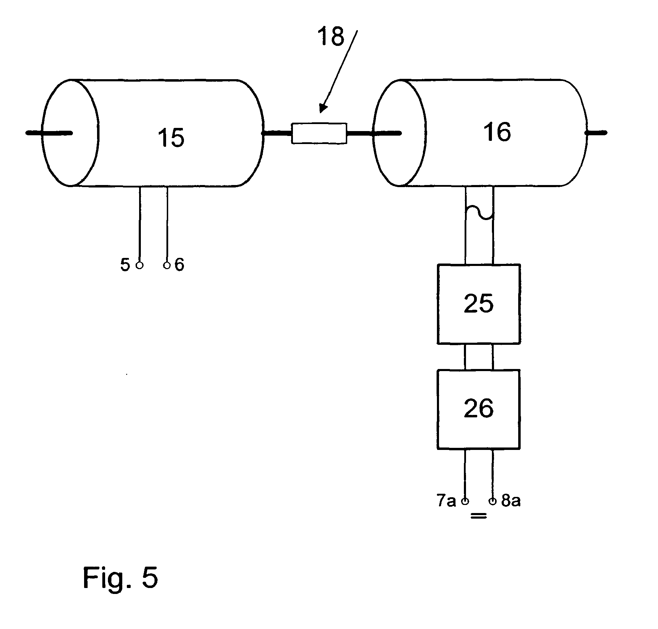

Auch wenn hier nur von einer Welle die Rede ist, so kann man die erfindungsgemäße Vorrichtung auch bilden, indem man die Welle des Motors mit der Welle des Generators mit einer gesonderten Verbindungswelle verbindet, wobei die gesonderte Welle aus isolierendem Material besteht und den elektrisch isolierenden Abschnitt bildet. Beispielsweise kann ein elektrisch isolierender Abschnitt 18 (Fig. 5) an die beiden Wellen des Motors bzw. Generators geflanscht werden. Es ist auch möglich, einen verbindenden Schlauch aus elektrisch isolierendem Material, auf die beiden Wellen von Motor und Generator zu ziehen, so dass das Drehmoment über den Schlauch übertragen wird. Die mechanische Kopplung der beiden Wellen kann auch über einen Riementrieb erfolgen, der zwei Riemenscheiben umfasst, die auf den jeweiligen Wellen befestigt sind, und einen auf diesen Riemenscheiben umlaufenden Riemen aus elektrisch isolierendem Material. Die Riemenscheiben können - zur Verringerung der elektrischen Kapazität - ebenfalls aus einem nichtleitenden Material geringster Dielektrizitätskonstante gefertigt sein.

Bei der erfindungsgemäßen Vorrichtung wird der Antriebsmotor mittels einer Energie- bzw. Spannungsquelle betrieben. Die Drehung seiner Welle wird elektrisch isoliert auf den Generator übertragen. Dieser erzeugt die zweite Spannung. Die zweite Spannung ist dann die Spannung, mittels derer die Messgeräte betrieben werden, die über Leitungen am menschlichen Körper, an oder in dem die Impedanz gemessen werden soll, angeschlossen werden.

Der Antriebsmotor kann ein Wechselstrommotor sein. Er kann allerdings auch ein Gleichspannungsmotor sein, mit Kollektor oder mit elektronischer Kommutierung, der über einen Gleichrichter oder aus einer Batterie betrieben wird. Es kann sich hier beispielsweise um eine herkömmliche Autobatterie handeln. Würde diese direkt mit den Messgeräten verbunden, so würde, da die Batterie in jedem Fall zumindest kapazitiv mit Erde (Masse) verkoppelt ist, die Impedanzmessung am Körper insbesondere bei höheren Frequenzen fehlerhaft werden.

Der Antriebsmotor muss nicht zwangsläufig ein Elektromotor sein;
z.B. wäre für einen speziellen Fall der Antrieb durch einen mit Druckluft betriebenen Motor (Turbine) möglich.

Der Generator kann ein Gleichspannungsgenerator sein, aber auch ein Wechselspannungsgenerator. In letzterem Fall dreht sich im Generator ein Permanentmagnet, der als Rotor fungiert, in einem ein- oder mehrphasigen Stator. Zweckmäßigerweise wird eine Dreiphasen-Wechselspannung erzeugt, da man nach Gleichrichtung einen Strom geringer Welligkeit erhält, der nur geringer Glättung (Einebnung der Welligkeit) bedarf. Auf die Verwirklichung der Kapazität von höchstens etwa 10 pF, insbesondere 5 pF, ist zu achten.

Ist die erste Spannung eine Netzspannung und die zweite Spannung eine Gleichspannung, ist die Vorrichtung nichts anderes als eine besondere Art von Netzteil.

Die erfindungsgemäße Kombination von Motor und Generator ist im Stand der Technik, wie eingangs ausgeführt, wohlbekannt, und zwar u.a. als rotierender Umformer oder Motor-Generator. Von dem erfindungsgemäßen Unterschied abgesehen, dass ein Abschnitt der Welle aus isolierendem Material sehr geringer Dielektrizitätskonstante besteht, geht es bei diesen Umformern jedoch üblicherweise um ganz andere Größenordnungen von Spannungen und insbesondere Leistungen, z. B. in Umformstationen. Bei der Vorrichtung nach der Erfindung ist die erste Spannung üblicherweise eine Netzwechselspannung von 230 V bzw. 110 V oder 100 V oder eine Batteriespannung von 12 V oder 24 V. Die zweite Spannung liegt in der Größenordnung von 5 bis 15 V Gleich- oder Wechselspannung. Das erfindungsgemäße Gerät ist auch wesentlich kleiner als die bekannten Leistungsumformer, da nur geringe elektrische Leistungen für die Messgeräte zur Verfügung zu stellen sind. Für eine Leistungsabgabe von 50 W können z.B. die längsten Abmessungen des Motor-Generator-Systems 20 cm betragen bei einem Durchmesser von weniger als 4 cm oder noch erheblich weniger, je nach der geforderten Ausgangsleistung.

Die Wellen oder die Verbindungswelle sollte nicht allzu kurz sein, da die Kapazität zwischen den metallischen Teilen der Eingangs- und Ausgangsseite möglichst unter 5 pF sein soll. Der freiliegende Bereich der Welle, d. h. der Bereich, der aus dem Teil des Motors vorsteht, der für den Antrieb der Welle verantwortlich ist und noch nicht in den Stator des Generators hineinragt, also der Teil, der außerhalb des (eigentlichen) Motors und des (eigentlichen) Generators liegt, sollte daher mindestens genauso lang wie der Motor oder der Generator sein, z. B. 5 bis 10 cm.

Eine lange Welle hat selbstverständlich nur dann Sinn, wenn der elektrisch isolierende Abschnitt möglichst lang ist. Bevorzugt ist der Abschnitt im wesentlichen so lang wie der Bereich der Welle, der außerhalb des Motors und des Generators liegt.

Eine lange Welle lässt sich mir modernen keramischen Werkstoffen verwirklichen. Eine Ausführungsform der Erfindung sieht daher vor, dass die Kopplungseinrichtung eine an ihren beiden Enden gelagerte keramische Welle ist, auf der - voneinander beabstandet - ein von einem Stator umgebener Motoranker und ein von einem Stator umgebener Generatoranker vorgesehen sind. Die Gehäuse der Statoren sind ausreichend weit voneinander entfernt, um die elektrischen Bedingungen einhalten zu können.

Da die erfindungsgemäße Vorrichtung in einem Raum eingesetzt wird, in dem Patienten behandelt werden, ist es von Vorteil, wenn die Vorrichtung möglichst leise arbeitet. Durch die Rotationsbewegung kann ein summendes Geräusch erzeugt werden. Vorzugsweise ist daher die Vorrichtung, die, wie oben erwähnt, nicht besonders groß sein muss, in ein schallabdichtendes Gehäuse eingebaut, d. h. in ein Gehäuse, das möglichst wenig Schall nach außen dringen lässt. Die Vorrichtung kann aber auch in das von ihr zu speisende Messgerät integriert werden.

Die erfindungsgemäße Vorrichtung zeichnet sich dadurch aus, dass sie eine hohe Potentialbarriere und einen hohen Isolationswiderstand zwischen Ein- und Ausgang hat, dass sie eine geringe elektrische Kapazität des Ausgangs (d.h. des Sekundärkreises) gegen Masse aufweist, dass primärseitige Störungen unterdrückt und selbst keine hochfrequente Störungen erzeugt werden. Kurze Unterbrechungen der Stromversorgung lassen sich insb. durch ein Schwungrad überbrücken. Sie eignet sich daher nicht nur zur Stromversorgung von Messgeräten in der Medizintechnik sondern auch in verwandten Bereichen wie der Biotechnik und der pharmazeutischen Technik.

Weitere Vorteile der Erfindung werden aus der nachfolgenden Beschreibung bevorzugter Ausführungsformen besser ersichtlich, die unter Bezug auf die beigefügte Zeichnung gegeben wird, welche schematisch erfindungsgemäße Vorrichtungen wiedergibt.

Es zeigen:
- Fig. 1: eine bekannte Vorrichtung zur Versorgung eines Impedanzmessgerätes gemäß dem Stande der Technik;
- Fig. 2: eine bekannte Vorrichtung ähnlich Fig. 1, bei der Transformatoren eingesetzt werden;
- Fig. 3: eine erste Ausführungsform der Erfindung;
- Fig. 4: ein zweite Ausführungsform der Erfindung;
- Fig. 5: eine dritte Ausführungsform der Erfindung mit ihren elektronischen Bauteilen;
- Fig. 6: eine vierte Ausführungsform der Erfindung mit ihren elektronischen Bauteilen, und
- Fig. 7: eine Abwandlung der Ausführungsform aus Fig. 6.

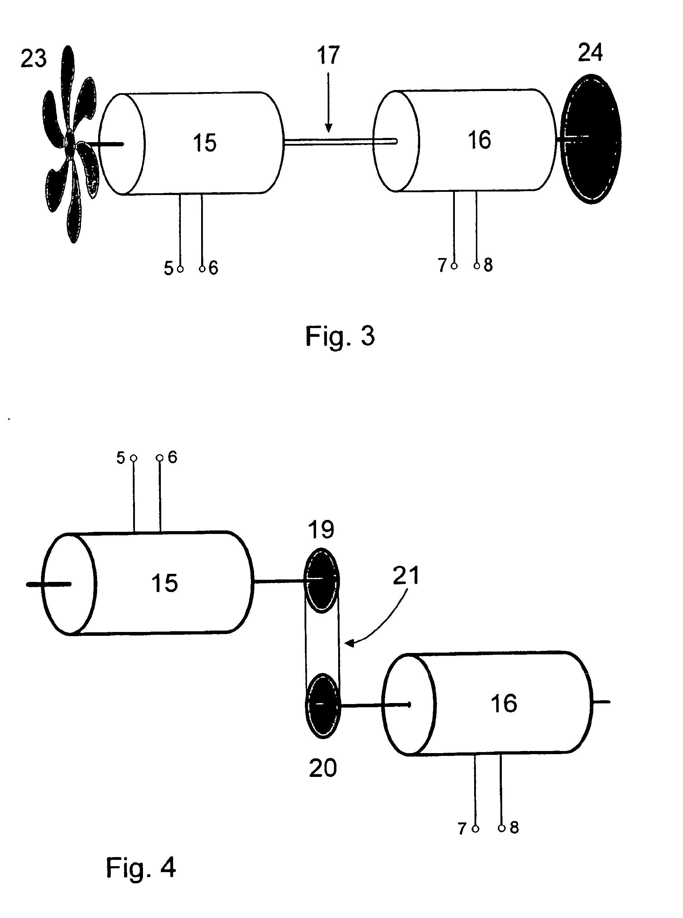

Bei der in Fig. 3 gezeigten ersten Ausführungsform der erfindungsgemäßen Stromversorgungsvorrichtung dreht ein von einer über die Anschlüsse 5 und 6 zugeführten ersten Spannung betriebener Antriebsmotor 15 über eine vollständig aus einem elektrisch isolierenden Material bestehende Welle 17 einen auf ihr sitzenden Rotor eines Generators 16, der die zweite Spannung erzeugt, welche über die Anschlüsse 7 und 8 des Generators zur Stromversorgung eines Messgeräts zur Verfügung steht.

Bei der in Fig. 4 dargestellten zweiten Ausführungsform ist ein Riementrieb 21 zwischen der üblichen, aus Welle aus Metall des Antriebsmotors 15 und der ebenfalls üblichen, aus Metall bestehenden Welle des Generators 16 vorgesehen. Auf beiden Wellenenden ist jeweils eine (aus Metall oder aus nichtleitendem Material gefertigte) Riemenscheibe 19 und 20 befestigt, über die ein isolierender Riemen 21 läuft.

Die erste Ausführungsform kann dahingehend abgewandelt werden, dass nicht die gesamte Welle 17 aus einem isolierenden Material besteht, sondern nur ein Abschnitt 18, wie dies bei der dritten Ausführungsform nach Fig. 5 dargestellt ist. In dieser Figur ist der Abschnitt 18 gegenüber der Welle verdickt gezeichnet, um eine qualitativ bessere Potentialtrennung darzustellen.

Auf der Motorwelle kann, z.B. auf einem nach außen verlängertem freien Wellenende, ein Lüfterrad 23, siehe Fig. 3, für eine forcierte Kühlung der Vorrichtung und/oder eines (integrierten) Messgerätes aufgebracht werden.

Kurzzeitige Netz-Einbrüche können durch ein Schwungrad 24 mit hinreichendem Trägheitsmoment auf einer verlängerten Motor- oder Generatorwelle überbrückt werden, s. Fig. 3.

Bei der in Fig. 5 dargestellten dritten Ausführungsform steht anstelle einer Wechselspannung - nach Fig. 3 kann je nach Generatorart eine Wechsel- oder Gleichspannung an den Anschlüssen 7 und 8 anliegen - nach einer Gleichrichtung in einem Gleichrichter 25 und einem Siebglied 26 eine Gleichspannung an den Anschlüssen 7a und 8a zur Verfügung.

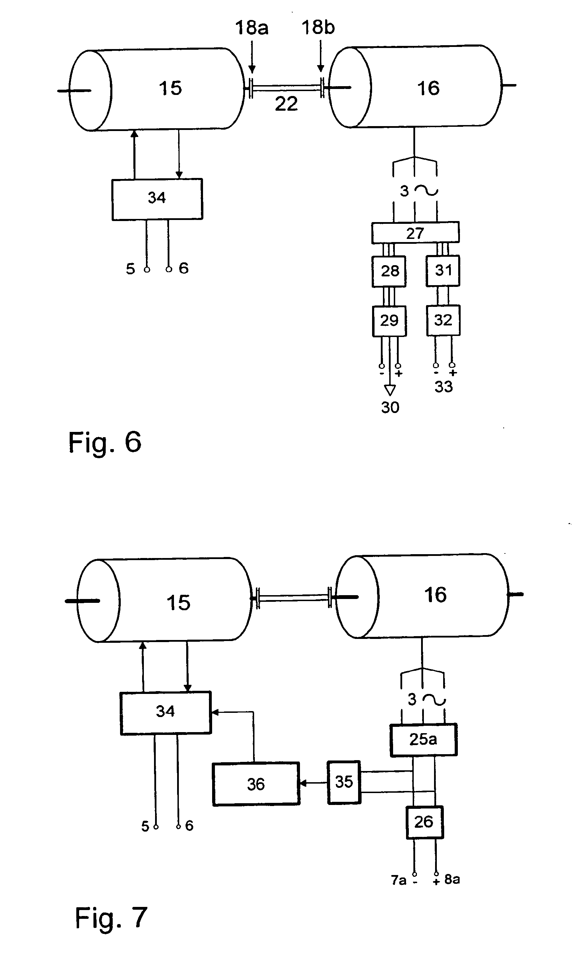

Die erfindungsgemäße Vorrichtung einer vierten Ausführungsform gemäß Fig. 6 umfasst als Hauptbestandteile einen (kollektorlosen) Gleichspannungsmotor 15, der z.B. von einer Autobatterie oder mit gleichgerichteter Spannung aus dem Netz betrieben werden kann, und einen Wechselspannungsgenerator 16. Der Antriebsmotor 15 treibt mit seiner Welle über eine, vorzugsweise flexible Kupplung 18a eine Zwischenwelle 22 aus isolierendem Material an, die über eine zweite, insb. ebenfalls flexible Kupplung 18b die Welle des Generators 16 antreibt. Hierfür wird bis auf die Zwischenwelle herkömmliche Technik verwendet. Der sich innerhalb des Motors 15 befindende Teil der Motorwelle trägt einen Permanentmagneten als Rotor.

Der sich innerhalb des Generators 16 befindende Abschnitt der Generatorwelle trägt ebenfalls einen Permanentmagneten und läuft in einem Dreiphasen-Stator.

Bei der Ausführungsform nach Fig.6 kann die Zwischenwelle 22 statt aus einem isolierenden Material auch aus leitendem Material, z.B. aus Metall, hergestellt sein. Dann aber müssen die flexiblen Kupplungen 18a und 18b aus isolierendem Material oder sonst isolierend bestehen.

Mit einer elastischen Ausbildung der Kupplungen lassen sich nicht zu vermeidende Fluchtungsfehler der Wellen von Motor- und Generator ausgleichen. Die Zwischenwelle 22 ist eine (nicht notwendigerweise) zylindrische Stange, d. h. sie hat normalerweise einen kreisförmigen Querschnitt.

Die isolierende Zwischenwelle 22 kann mit Vorteil aus Trovidur®, d. h. einem elektrisch isolierenden Material, gefertigt sein. Sie hat etwa die Länge wie die aus dem Motor und dem Generator herausragenden Wellenabschnitte.

In einer anderen Ausführung sind die Kupplungen 18a und 18b und die isolierende Zwischenwelle 22 oder der isolierende Abschnitt 18 der Welle 17 durch einen auf die Wellenenden von Motor und Generator aufgezogenen elastischen Schlauch ersetzt, s. Fig. 5.

Die Generatorspannung wird über einen Drei-Phasen-Transformator 27 (Fig. 6) mit zwei getrennten Sekundärwicklungen zwei verschiedenen Gleichrichtern 28 und 31 und Siebgliedern und Spannungsstabilisatoren 29 und 32 zugeführt, wobei an den Anschlüssen 30 des Spannungsstabilisators 29 Spannungen von +15 V und -15 V gegenüber dem Nullpunkt der Ausgangsseite entnehmbar sind, und an den Anschlüssen 33 des Spannungsstabilisators 32 eine unabhängige Spannung von 5 V entnehmbar ist.

Die erfindungsgemäße Vorrichtung ist sehr klein; z.B. für eine Leistung von ca. 50 W hat der Antriebsmotor eine Länge von 6 cm, und der Durchmesser des hier zylindrisch gestalteten Motors beträgt 3,2 cm. Der Generator hat im wesentlichen dieselben Maße. Der außerhalb des Motors 15 und des Generators 16 liegende, in der Figur freiliegende (also gewissermaßen "an der Luft" liegende) Abschnitt der Welle hat eine Länge von etwa 10 cm. Die isolierenden Welle 22 hat eine Länge von ca. 5 cm.

Der Antriebsmotor 15 (Fig. 6) kann auch mit einer Motorsteuerung 34 ausgerüstet werden, die die Drehzahl der Vorrichtung unabhängig von Schwankungen der Versorgungsspannung und der Last konstant hält.

Bei zu erwartenden größeren Schwankungen der Last kann, wie dies für eine abgewandelte Ausführungsform in Fig. 7 dargestellt ist, die Ausgangsspannung des Gleichrichters 25a über beispielsweise einen linearen Optokoppler 35 abgenommen und mittels eines zusätzlichen Reglers 36 auf einem konstanten Wert gehalten werden.

Außerdem kann der Regler 36 Überlast erkennen und signalisieren und gegebenenfalls den Antriebsmotor 15 abschalten.

Wegen der kleinen Abmessungen der Bauteile kann die gesamte Vorrichtung mit dem zu speisenden Messgerät in ein Gehäuse integriert oder in einem kleinen schalldämmenden Gehäuse untergebracht werden, das zwar Lüftungsschlitze aufweist, damit die von dem Motor und dem Generator erzeugte Wärme entweichen kann, aber dennoch für eine Schalldämpfung sorgen kann.

Es ist möglich, sowohl für den Motor als auch für den Generator aus Kostengründen den gleichen Typ eines kollektorlosen Motors einzusetzen, der im Prinzip aus einer dreiphasigen Synchronmaschine mit permanentmagnetisiertem Läufer besteht. Die Lebensdauer dieser Art Maschinen ist nur noch durch die ihrer Lager begrenzt.

## Patentansprüche

1. Vorrichtung zum potentialgetrennten Umwandeln einer ersten Spannung in eine zweite Spannung zum Messen von Impedanzen und Admittanzen an biologischen Geweben, insbesondere am menschlichen Körper, mittels eines aktiven elektronischen Messgeräts
mit
einem mit der ersten Spannung betriebenen Antriebsmotor (15),
einem von dem Motor angetriebenen Generator (16), **dadurch gekennzeichnet, dass** der Generator (16) die zweite Spannung unabhängig von der Masse der ersten Spannung erzeugt, und **dass** die Vorrichtung mindestens einer Kopplungseinrichtung zwischen Motor und Generator aus einem elektrisch isolierenden Kunststoffmaterial wie Nylon, Trovidur® (Markenname) oder Polystyrol oder einem Keramikmaterial wie Degussit® (Markenname) oder Zirkonoxid und einer solchen Abmessung, dass die Kapazität des Generators gegen Masse höchstens etwa 10 pF, insb. 5 pF, beträgt, aufweist.

2. Vorrichtung nach Anspruch 1, bei der die Kopplungseinrichtung wenigstens eine Welle (17, 22) oder eine Zwischenwelle (18) ist, die wenigstens zu einem Teil (18) aus dem elektrisch isolierenden Material besteht.

3. Vorrichtung nach Anspruch 2, bei der der Abschnitt (18) aus isolierendem Material im wesentlichen so lang wie der Bereich der Welle ist, der außerhalb des Antriebsmotors (15) und des Generators (16) liegt.

4. Vorrichtung nach Anspruch 3, bei der die Kopplungseinrichtung eine Zwischenwelle (22) und zwei Kupplungen (18a, 18b) für die Verbindung der Welle des Antriebsmotors (15) mit der Welle des Generators (16) aufweist, und wenigstens eines dieser Bauteile (18a, 18b, 22) aus dem elektrisch isolierendem Material gefertigt ist.

5. Vorrichtung nach Anspruch 4, bei der die Kopplungseinrichtung eine an ihren beiden Enden gelagerte keramische Welle ist, auf der voneinander beabstandet ein von einem Stator umgebener Motoranker und ein von einem Stator umgebener Generatoranker vorgesehen sind.

6. Vorrichtung nach einem der vorhergehenden Ansprüche, bei der die längste Abmessung (I) des Antriebsmotors (15) 20 cm, vorzugsweise 10 cm, und/oder die längste Abmessung des Generators (16) 20 cm, vorzugsweise 10 cm beträgt und/oder der aus dem Antriebsmotor (15) und dem Generator (16) herausragende Bereich der Welle, 10 cm, vorzugsweise 5 cm, besonders bevorzugt 3 cm lang ist.

7. Vorrichtung nach einem der Ansprüche 1 bis 6, deren Generator (16) einen permanentmagnetischen Rotor und einen Drei-Phasen-Stator, der eine Drei-Phasen-Wechselspannung als zweite Spannung erzeugt, hat.

8. Vorrichtung nach einem der Ansprüche 1 bis 7, bei der eine Motorsteuerung (34) zur Konstanthaltung der Drehzahl des Antriebsmotors (15) vorgesehen ist.

9. Vorrichtung nach einem der Ansprüche 1 bis 8, bei der für die im Generator (16) erzeugte ein- oder mehrphasige Wechselspannung ein Gleichrichter (25; 25a; 28, 31), eine Siebeinrichtung (26) und eine Stabilisierungseinrichtung (29, 32) vorgesehen sind, die die resultierende Spannung unabhängig von der Masse der Eingangsspannung erzeugen.

10. Vorrichtung nach Anspruch 9, bei der ein Regler (36) zur Konstanthaltung der Ausgangsspannung des Gleichrichters (25a) und ein Optokoppler (35) als potentialtrennendes Element (35) zwischen dem Ausgang des Gleichrichters (25a) und dem Eingang des Reglers (36) vorgesehen sind.

11. Vorrichtung nach einem der vorhergehenden Ansprüche, bei der das Motor-Generator-Aggregat, vorzugsweise auf der Motorachse, ein Lüfterrad (23) ausweist.

12. Messgerät zum Messen von Impedanzen und Admittanzen an biologischen Geweben, mit einem Gehäuse und einer Vorrichtung nach einem der vorhergehenden Ansprüche, die in das Gehäuse des zu speisenden Messgerätes integriert ist.

## Claims

1. Apparatus for isolated transformation of a first voltage into a second voltage for measurement of electrical impedances or admittances, respectively, of biological tissues, in particular of the human body, by means of an active electronic measuring device including a motor (15) operated by the first voltage and a generator (16) driven by said motor,
**characterized in that**
the generator (16) generates the second voltage independent of the ground potential of the first voltage, and **in that** the apparatus features at least one coupling means arranged between the motor and the generator made of electrically isolating plastic material such as nylon, Trovidur® (trademark) or polystyrene or of a ceramic material such as Degussit® (trademark) or zirconium dioxide (ZrO₂), and of such dimension that the capacitance of the generator with reference to ground is not more than 10 pF, especially 5 pF.

2. Apparatus according to claim 1 wherein the coupling means consists of at least a shaft (17, 22) or an intermediate shaft (18), at least a portion (18) thereof being made of an electrically isolating material.

3. Apparatus according to claim 2 wherein the portion of the intermediate shaft (18) made of isolating material is approximately as long as the portion of the shaft external to the motor (15) and the generator (16).

4. Apparatus according to claim 3 wherein the coupling means features an intermediate shaft (22) and two couplings (18a, 18b) for connection of the motor shaft (15) with the generators shaft (16), and wherein at least one of these components (18a, 18b, 22) is made of an electrically isolating material.

5. Apparatus according to claim 4 wherein the coupling means is a ceramic shaft with bearings supporting its ends, on which both a motor anchor surrounded by its stator and, in some distance, a generator anchor surrounded by its stator, are attached.

6. Apparatus according to one of the aforementioned claims wherein the largest dimension of the motor (15) is 20 cm, preferably 10 cm, and/or the largest dimension of the generators (16) is 20 cm, preferably 10 cm, and/or the shaft extending beyond the motor (15) and the generator (16) is 10 cm, preferably 5 cm, or more advantageously 3 cm.

7. Apparatus according to any of claims 1 to 6 wherein the generator (16) incorporates a permanent magnetic rotor and a three-phase stator, which generates a three/phase alternating voltage as the second voltage.

8. Apparatus according to any of claims 1 to 7 wherein a motor control means (34) is provided for keeping the speed of the motor (15) constant.

9. Apparatus according to any of claims 1 to 8 wherein for a single- or multiple phase alternating voltage generated by the generator (16), a rectifier (25; 25a; 28, 31), a filter (26) and a stabilizing means (29, 32) are provided, which generate a resulting voltage independent of the ground potential of the first (input) voltage.

10. Apparatus according to claim 9, which includes a control means (36) for keeping the output voltage of the rectifier (25a) at a constant level, and an optocoupler means(35) as a potential-isolating element (35) between the output of the rectifier (25a) and the input of the control (36).

11. Apparatus according to any of the aforementioned claims wherein the motor-generator employs a propeller (23) preferably mounted onto the motor shaft.

12. Measuring device for measuring of impedances or admittances of biological tissues build into an enclosure which incorporates an apparatus according to any of the aforementioned claims.

## Revendications

1. Dispositif pour la transformation isolée d'une première tension en une seconde tension pour la mesure des impédance ou admittance électriques de tissus biologiques, en particulier du corps humain, par le biais d'un appareil de mesure électronique active comprenant un moteur (15) commandé par la première tension et un générateur (16) entraîné par ledit moteur, **caractérisé en ce que**
le générateur (16) génère la deuxième tension de manière indépendante de la terre de la première tension, et que l'appareil possède au moins un dispositif de couplage, monté entre le moteur et le générateur, fait d'un matériau isolant tel que le Nylon,
le Trovidur® (marque déposée) ou le polystyrène ou fait d'un matériau céramique tel que le Degussit® (marque déposée) ou le dioxyde de zirconium (ZrO₂), et de dimension telle que la capacité électrique du générateur ne dépasse pas, par rapport à la terre, 10 pF, en particulier 5 pF.

2. Dispositif selon la revendication 1, **caractérisé en ce que** le dispositif de couplage consiste en au moins un axe (17, 22) ou un axe intermédiaire (18), dont une partie, au moins, (18) est en matériau isolant.

3. Dispositif selon la revendication 2, **caractérisé en ce que** la partie de l'axe intermédiaire (18) faite de matériau isolant est à peu près aussi longue que la partie de l'axe se trouvant à l'extérieur du moteur (15) et du générateur (16).

4. Dispositif selon la revendication 3, **caractérisé en ce que** le dispositif de couplage est un axe intermédiaire (22) et deux couplages (18a, 18b) pour connecter l'axe du moteur (15) et l'axe du générateur (16), et où, au moins un de ces composants (18a, 18b, 22) est fait de matériau isolant.

5. Dispositif selon la revendication 4, **caractérisé en ce que** le dispositif de couplage est un axe de céramique comportant, à chaque bout, des coussinets, sur lesquels sont fixés la pièce d'ancrage du moteur entouré de son stator et, à quelque distance, la pièce d'ancrage du générateur entouré de son stator.

6. Dispositif selon l'une quelconque des revendications précédentes, **caractérisé en ce que** la plus grande dimension du moteur (15) est de 20 cm, de préférence 10 cm, et/ou la plus grande dimension du générateur (16) est de 20 cm, de préférence 10 cm, et/ou la partie de l'axe dépassant du moteur (15) et du générateur (16) est de 10 cm, de préférence 5 cm, ou de manière encore plus avantageuse de 3 cm.

7. Dispositif selon l'une quelconque des revendications 1 à 6, **caractérisé en ce que** le générateur (16) comprend un rotor magnétique permanent et un stator à trois phases, qui génère une tension à trois phases comme seconde tension.

8. Dispositif conforme à l'une des revendications 1 à 7, **caractérisé en ce que** une entité de contrôle du moteur (34) est incorporée de manière à maintenir la vitesse du moteur (15) constante.

9. Dispositif selon l'une quelconque des revendications 1 à 8, **caractérisé en ce que**, pour la tension alternative de phase simple ou multiple qui est engendrée par le générateur (16), sont fournis un redresseur (25; 25a; 28, 31), un filtre (26) et un moyen de stabilisation (29, 32) qui génère une tension indépendante de la terre de la première tension (donnée d'entrée).

10. Dispositif selon la revendication 9, **caractérisé en ce que** sont fournis une entité de contrôle (36) de manière à maintenir constante la tension de sortie du redresseur (25a), et un optocoupleur (35) en tant qu'élément isolant par rapport au potentiel (35) entre la sortie du redresseur (25a) et l'entrée de l'entité de contrôle (36).

11. Dispositif selon l'une quelconque des revendications précédentes, **caractérisé en ce que** l'entité moteur générateur emploie une hélice (23) montée de préférence sur l'axe du moteur.

12. Dispositif de mesure pour la mesure d'impédances ou d'admittances de tissus biologiques incorporé dans un boîtier comprenant un dispositif conforme à l'une des revendications précédentes.
